# EUROPÄISCHE PATENTSCHRIFT

(11) **EP 2 768 401 B1**
(45) Veröffentlichungstag und Bekanntmachung des Hinweises auf die Patenterteilung: **29.11.2017**
(21) Anmeldenummer: 13700877.7
(22) Anmeldetag: 15.01.2013
(51) Int. Cl.: A61B 17/16, A61B 17/84, A61B 17/88

(54) **MTV-IMPLANTATIONS-SET**
MTV IMPLANTATION SET
KIT D'IMPLANTATION MTV

(43) Veröffentlichungstag der Anmeldung: 27.08.2014
(73) Patentinhaber: Müller, Friedrich, 21337 Lüneburg (DE)
(72) Erfinder: Müller, Friedrich, 21337 Lüneburg (DE)
(74) Vertreter: Knoop, Philipp
(86) Internationale Anmeldenummer: PCT/EP2013/050682
(87) Internationale Veröffentlichungsnummer: WO 2014/111134

(56) Entgegenhaltungen:
- WO-A2-2011/031495
- US-A1- 2011 087 296
- US-A1- 2011 118 792

## Beschreibung

Die vorliegende Offenbarung betrifft ein orthopädisches Implantat zum dauerhaften Befestigen an Knochen im Körper von Wirbeltieren und/oder Menschen, insbesondere um damit Wirbel zu verblocken, mit einem länglichen, im Wesentlichen zylindrischen Grundkörper.

Die vorliegende Offenbarung-betrifft ferner eine Führungsvorrichtung zum Vorbohren eines im Wesentlichen zylindrischen Kanals im Knochen im Körper von Wirbeltieren und/oder Menschen.

Ferner betrifft die vorliegende Offenbarung Kontermittel zum Zusammenwirken mit dem Konterabschnitt eines Implantats
gemäß eines Implantats des vorgenannten Satzes sowie Anschlussmittel mit einem Abschnitt zur Herstellung einer Wirkverbindung mit einer Bohrvorrichtung.

Die vorliegende Erfindung betrifft einen Satz umfassend mindestens ein Implantat, mindestens eine Führungsvorrichtung, mindestens ein Kontermittel sowie mindestens ein Anschlussmittel mit einem Abschnitt zur Herstellung einer Wirkverbindung mit einer Bohrvorrichtung.

Ein orthopädisches Implantat der eingangs genannten Art kann bekanntermaßen im einfachsten Fall ein sogenannter Steinmann-Nagel sein. Damit lassen sich beispielsweise in der Neurochirurgie, besonders im veterinärmedizinischen Bereich, Behandlungen des sogenannten Cauda-Equina-Syndroms (CES), der degenerativen lymbosakralen Stenose (DLSS) oder der Folgen der degenerativen Discopathie zwischen Lendenwirbelsäule und Kreuzbein behandeln. Gemäß einer von dem Erfinder der vorliegenden Erfindung entwickelten Operationsmethode kann mit Hilfe eines gattungsgemäßen Implantats, also herkömmlich mit einem Steinmann-Nagel, in vielen Fällen eine Vertebralverblockung minimal invasiv und ambulant, das heißt insbesondere ohne große Durchtrennung von Gewebe wie Haut, Muskulatur oder Knochen, durchgeführt werden. Dabei verhindert die Verblockung ein Zurück- und Aufwärtsgleiten des letzten Lendenwirbels, die sogenannte Subluxation, und dadurch ein Einklemmen der Nervenwurzeln. Außerdem kann durch die Verblockung die Bandscheibe druckentlastet und bereits bestehende Vorwölbungen oder Vorfälle vermindert werden. Im Prinzip wird der bekannte Steinmann-Nagel bei der transilialen Vertebralverblockung insbesondere seitlich durch die Hüftknochen geführt, um die gewünschte Verblockung zu erreichen.

Bei der Verwendung von Steinmann-Nägeln zu dem genannten Zweck ist es gemäß dem Stand der Technik jedoch mit Nachteil erforderlich, zur Fixierung des Implantats im Körper das Implantat, also den Steinmann-Nagel, während der Operation unmittelbar am Einsatzort, also am Knochen des Patienten, in geeigneter Weise zu biegen und vor Ort in geeigneter Weise zu kürzen. Sowohl der Biegevorgang als auch der Kürzungsvorgang des Nagels während der OP im Körper ist dabei jedoch mit Nachteil problematisch. Er führt häufig zu unerwünschter Traumatisierung des umgebenden Gewebes und kann zudem im ungünstigsten Fall auch nachhaltig den Heilungsprozess erschweren.

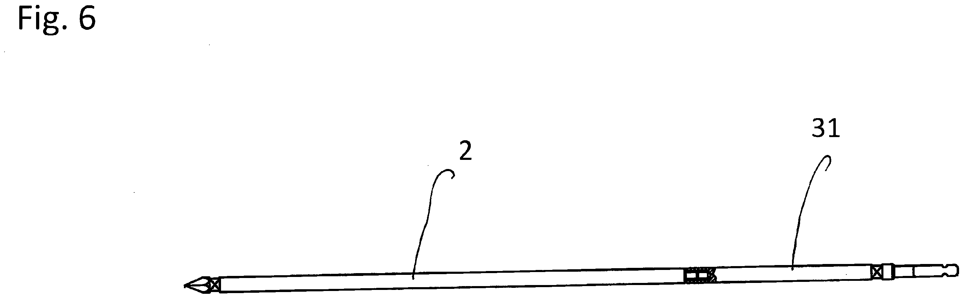

Die WO 2011/031495 A2 offenbart ein Knochen-Kompressionssystem. Insbesondere ist in den Figuren 6a bis 6i eine Knochenschraube und deren Verwendung im Zusammenhang mit der Fixierung von Finger- oder Zehenknochen veranschaulicht. Bei dem in den Figuren 6a und 6b zu erkennenden Gegenstand handelt es sich um ein orthopädisches Implantat zum dauerhaften Befestigen im Knochen im Körper von Menschen mit einem länglichen, im Wesentlichen zylindrischen Grundkörper, welcher ein Fixierungsaußengewinde zum Eindrehen in den Knochen aufweist.

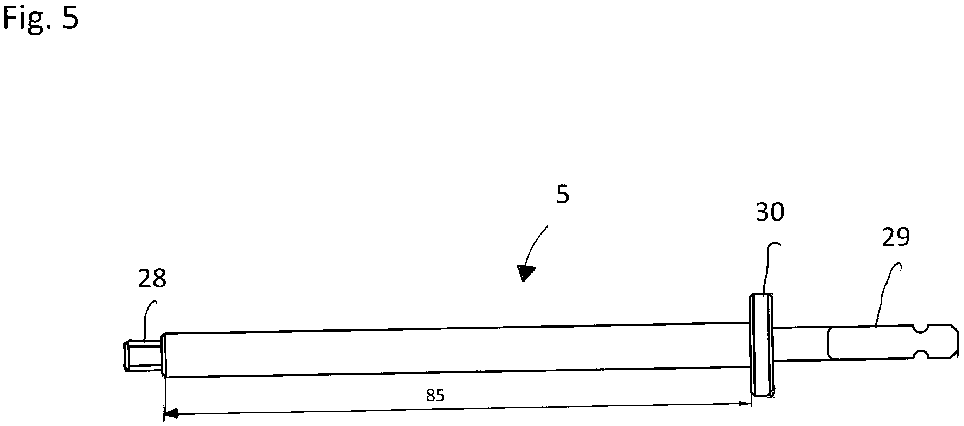

Aus der US 2011/0118792 A1 ist ein Verfahren und Vorrichtung zum Einführen eines orthopädischen Fixierungsnagels mit einem Blindloch bekannt. Wie in den Figuren 5 und 6, die einen derartigen Fixierungsnagel zeigen, sowie in den Figuren 14 und 15, welche die Verwendung des in den Figuren 5 und 6 gezeigten Nagels am Knochen zeigen, zu erkennen, weist der bekannte Nagel ein Fixierungsaußengewinde 222 auf, welches zwei komplette Gewindegänge umfasst und dessen Durchmesser größer als jener der Knochenschraube ist.

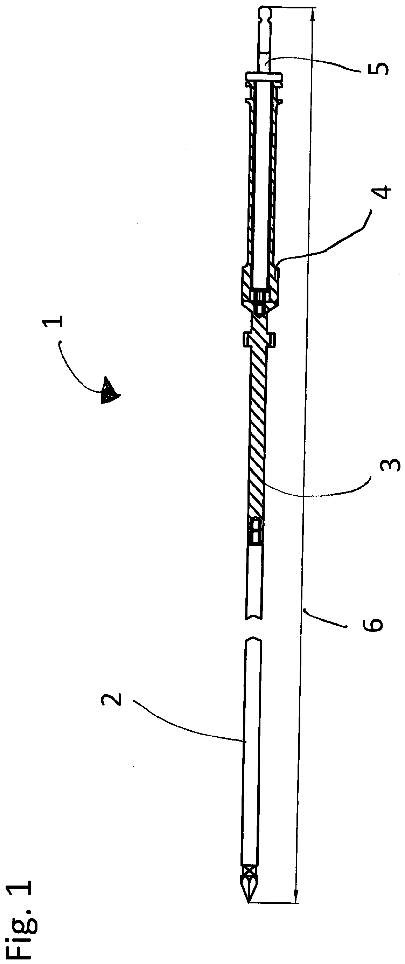

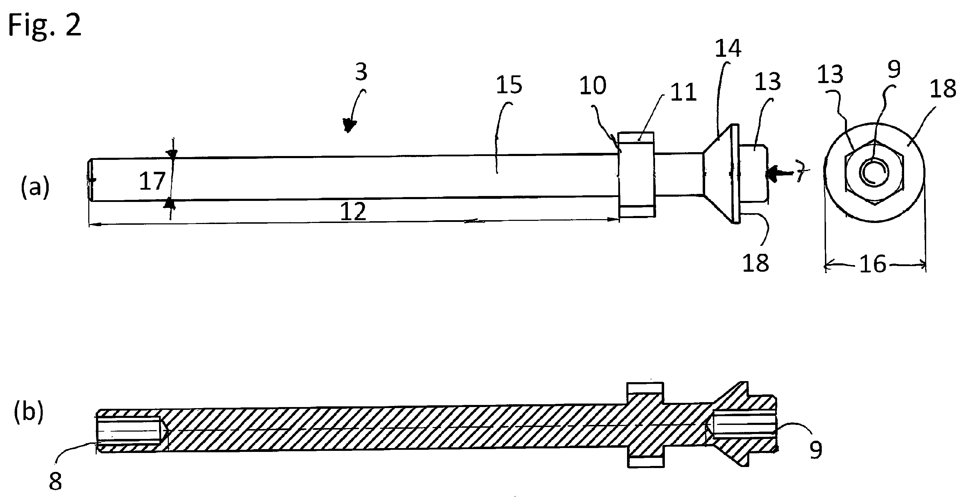

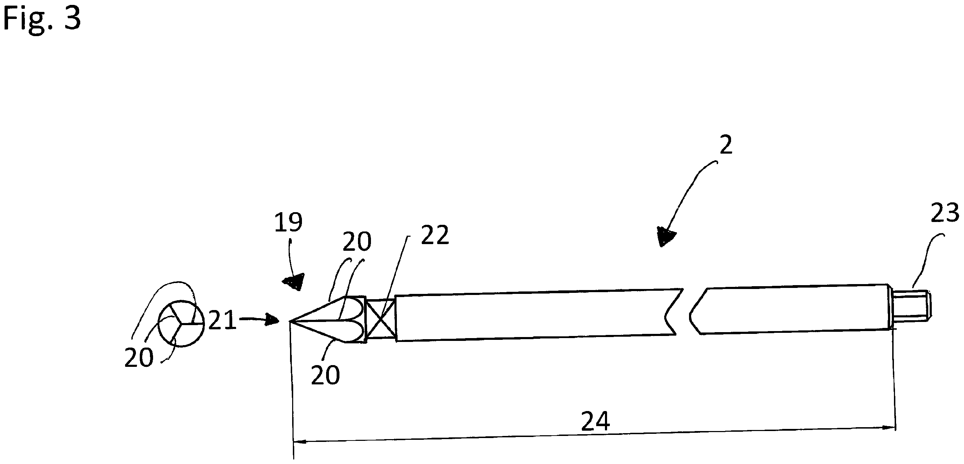

Aus der Offenlegungsschrift US 2011/0087296 A1 sind Systeme und Verfahren zur Fixierung von Knochenverbindungen unter Verwendung von kompressiblen Implantaten bekannt. Wie in der Fig.1 in Verbindung mit Fig.2 zu erkennen, besteht das bekannte orthopädische Implantat, welches zum dauerhaften Befestigen am Knochen in Körper von Wirbeltieren und/oder Menschen vorgesehen ist, aus einem länglichen Grundkörper, welcher in der Ausgestaltung gemäß Fig.3 jedenfalls als im wesentlichen zylindrisch angesehen werden kann. Wie in den Figuren 1 und 2 zu erkennen, weist der Grundkörper ein Fixierungsaußengewinde 14, 30 auf, welches zum Eindrehen in den Knochen bestimmt ist.

Vor diesem Hintergrund liegt der vorliegenden Erfindung die Aufgabe zugrunde, einen Satz der eingangs genannten Art anzugeben, umfassend ein orthopädisches Implantat, welches durch eine minimal-invasive Behandlung in den Patienten unter geringer Traumatisierung des umgebenden Gewebes eingebracht werden kann, um Heilungserfolg und Genesungsdauer nachhaltig zu verbessern.

Mit Bezug auf das orthopädische Implantat zum dauerhaften Befestigen an Knochen im Körper von Wirbeltieren und/oder Menschen, insbesondere um damit Wirbel zu verblocken, mit einem länglichen, im Wesentlichen zylindrischen Grundkörper, wird diese Aufgabe dadurch gelöst, dass ein
Implantat ein Fixierungsaußengewinde zum Einbringen in den Knochen aufweist. Die Fixierung des Implantats mittels eines Gewindes im Knochen hat den Vorteil, dass eine Biegung des Implantats *in vivo* nicht erforderlich ist, um beispielsweise eine Verblockung zu erzielen, welche ein Zurück- und Aufwärtsgleiten des letzten Lendenwirbels verhindert.

In vorteilhafter Ausgestaltung des Implantats gemäß dem erfindungsgemäßen Satz umfasst das Fixierungsaußengewinde zwei komplette Gewindegänge. Diese Ausgestaltung ermöglicht eine sichere, dauerhafte Befestigung des Implantats im Knochen.

Insbesondere ist es erfindungsgemäß von Vorteil, wenn das Fixierungsaußengewinde einen größeren Durchmesser als der Grundkörper aufweist und/oder wenn das Fixierungsaußengewinde in einem wulstartig erweiterten Abschnitts des Grundkörpers ausgeformt ist. Das Fixierungsaußengewinde hat dabei erfindungsgemäß einen größeren Durchmesser als der zylindrische Grundkörper des erfindungsgemäßen Implantats. Dadurch ist es etwa möglich, das Fixierungsaußengewinde in der Nähe eines Endabschnitts des zylindrischen Grundkörpers anzuordnen, um das Implantat gemäß dem erfindungsgemäßen Satz an einem Hüftknochen zu fixieren und im Übrigen den dünneren zylindrischen Grundkörper durch den Hüftknochen hindurch zu stecken.
Im Rahmen der Erfindung ist es zudem vorteilhaft, wenn bei dem

Implantat gemäß dem erfindungsgemäßen Satz der Grundkörper an einem vorderen Ende mit vorderen Befestigungsmitteln, insbesondere einem vorderen Innengewinde, zur lösbaren Befestigung einer Führungsvorrichtung in Verlängerung des Grundkörpers versehen ist. Auf diese Weise kann, in etwa vergleichbar mit bestimmten Verfahren im Tunnelbau, in einem kontinuierlichen Arbeitsgang eine gewünschte Bohrung im Knochen erstellt werden, in welche beim Vortrieb der Führungsvorrichtung aufgrund deren Befestigung am Implantat automatisch das Implantat eingeführt wird.

Weiterhin ist es erfindungsgemäß vorteilhaft, wenn das Implantat in Ausgestaltung des Satzes gemäß der Erfindung an einem hinteren Ende mit hinteren Befestigungsmitteln, insbesondere einem hinteren Innengewinde, zur lösbaren Befestigung von zur Herstellung einer Wirkverbindung mit einer Bohrvorrichtung ausgeformten Anschlussmitteln versehen ist. Im Rahmen des Vorliegenden beziehen sich die Begriffe "vorderes Ende" bzw. "hinteres Ende" auf die Vortriebsrichtung beim Einsetzen des Implantats in den Körper. Entscheidend ist dabei die Lösbarkeit der Verbindung. Denn diese ermöglicht es, beispielsweise durch Lösen eines Gewindes die Führungsvorrichtung zu entfernen, so dass das Implantat in einer vorkonfektionierten Länge im Körper verbleiben kann, nachdem es eingesetzt wurde.

Des Weiteren ist in einer bevorzugten Ausgestaltung des
Implantats gemäß dem erfindungsgemäßen Satz vorgesehen, dass dieses an einem hinteren Ende mit hinteren Befestigungsmitteln, insbesondere einem hinteren Innengewinde, zur lösbaren Befestigung von zur Herstellung einer Wirkverbindung mit einer Bohrvorrichtung ausgeformten Anschlussmitteln versehen ist. Auch hier wird mit Vorteil die lösbare Verbindbarkeit des Implantats mit beispielsweise einem Bohraufsatz mit standardisierten AO-Schaft ermöglicht. Dieser kann in übliche Bohrfutter eingesetzt werden, um das Implantat an der gewünschten Stelle einzusetzen und im Knochen zu verankern. Sobald jedoch die gewünschte Position des Implantats im Knochen erreicht ist, können die Anschlussmittel vom Implantat gelöst werden. Dies erfolgt im einfachsten Falle, indem sie schlicht abgeschraubt werden. Auch hier liegt der Vorteil darin, dass das Implantat in der für die Erzielung der medizinischen Wirkung gewünschten Länge vorkonfektioniert sein kann und für den Einsetzvorgang ein lösbarer Bohraufsatz mit AO-Schaft genutzt werden kann, welcher das Implantat lediglich während des Einsetzvorgangs effektiv verlängert, um es von außerhalb des Körpers eindrehen zu können.

In vorteilhafter Ausgestaltung des Satzes nach der Erfindung ist bei dem Implantat vorgesehen, dass der Grundkörper mindestens einen, vorzugsweise zwischen dem Fixierungsaußengewinde und dem hinteren Ende angeordneten, Konterabschnitt mit einem nicht-kreisförmigen Querschnittsprofil aufweist. Um die Traumatisierung des umgebenden Gewebes möglichst gering zu halten, und um ein in dem Knochen mittels des Fixierungsaußengewindes in gewünschter Position fixiertes Implantat nicht mehr in seiner Position zu verändern, lässt sich mit Hilfe des Konterabschnitts beim Entfernen der Führungsvorrichtung bzw. der Anschlussmittel für die Bohrvorrichtung sicherstellen, dass zum Beispiel keine Drehmomente auf das Implantat beim Lösen der Schraubverbindungen übertragen werden. Der Konterabschnitt im Sinne der Erfindung kann beispielweise in Form einer Sechskantmutter geformt sein bzw. indem die Peripherie des Implantats im Bereich des Konterabschnitts die Form einer Sechskantmutter aufweist.

Um bei der Benutzung des Konterabschnitts beim Entfernen der Führungsvorrichtung bzw. der Anschlussmittel für den Bohrer sicherzustellen, dass durch das Kontern keine Traumatisierung des Gewebes erfolgt, ist in Ausgestaltung des Implantats gemäß dem erfindungsgemäßen Satz vorgesehen, dass der Grundkörper einen sich an den Konterabschnitt, vorzugsweise in Richtung des vorderen Endes, anschließenden, diesen an dem ihn zugewandten Ende radial überragenden und sich, vorzugsweise in Richtung des vorderen Endes, verjüngenden konischen Abschnitt aufweist. Die Grundfläche des konischen Abschnitts am hinteren Ende fungiert dabei als Anlagefläche, der konische Abschnitt stellt hingegen einen möglichst glatten Übergang in Richtung des Knochens und der Schraubverbindung sicher.

Mit Vorteil weisen in Ausgestaltung der Erfindung das vordere Innengewinde und das hintere Innengewinde des Implantats des erfindungsgemäßen Satzes eine übereinstimmende Drehrichtung auf. Beispielsweise können standardmäßige Rechtsgewinde zum Einsatz kommen. Wenn das Implantat gemäß dem erfindungsgemäßen Satz mit einem Konterabschnitt, etwa einer Sechskantmutter, versehen ist, kann das Lösen der Schraubverbindung nachdem das Implantat die gewünschte Position im Knochen erreicht hat, in jedem Fall erfolgen, ohne dass weitere Momente auf das Implantat übertragen werden. Deswegen ist beispielsweise auch keine gegenläufige Ausgestaltung der Gewinde erforderlich, um Drehmomente vom Implantat fernzuhalten.

Mit Blick auf die gegenständliche Führungsvorrichtung zum Vorbohren eines im Wesentlichen zylindrischen Kanals im Knochen im Körper von Wirbeltieren und/oder Menschen wird die eingangs genannte Aufgabe dadurch gelöst, dass die Führungsvorrichtung zur lösbaren Befestigung in Verlängerung des Grundkörpers eines Implantats
der vorgenannten Art durch Zusammenwirken mit den vorderen Befestigungsmitteln ausgebildet ist, wobei die Führungsvorrichtung mindestens einen Konterabschnitt aufweist, welcher ein nichtkreisförmiges Querschnittsprofil aufweist. Der Konterabschnitt kann im Rahmen der Erfindung als Vierkantabschnitt ausgestaltet sein, mit welchem bei gekontertem Implantat durch Einsatz eines entsprechenden Vierkantschlüssels die Führungsvorrichtung vom Implantat gelöst, beispielsweise abgeschraubt, werden kann.

Vorzugsweise hat in Ausgestaltung der Erfindung die Führungsvorrichtung an einem hinteren Ende ein zu dem vorderen Innengewinde des Implantats komplementäres Außengewinde. Auf diese Weise kann mit Vorteil die Führungsvorrichtung, welche zum Beispiel als Führungsnagel zum Vorbohren ausgestaltet sein kann, vor dem Einsetzen des Implantats mit dem Implantat verschraubt werden.

Wenn in Ausgestaltung der Führungsvorrichtung gemäß dem erfindungsgemäßen Satz ein vorderes Ende, vorzugsweise nach Art einer geraden Pyramide, spitz zulaufend ausgeformt ist, mit zum Schneiden und/oder Bohren von Knochen ausgebildeten Kanten, lässt sich die Führungsvorrichtung nach Art eines Bohrers verwenden, welcher beim Herstellen der Bohrung das Implantat in die erstellte Bohrung zieht, wenn das Implantat mit dem auf diese Weise ausgestalteten Führungsnagel verschraubt ist. Die spezielle Ausformung des Endes nach Art einer Pyramide hat sich erfindungsgemäß als vorteilhaft hinsichtlich der Minimierung einer Traumatisierung des angrenzenden Gewebes erwiesen.

Mit Blick auf die Kontermittel der eingangs genannten Art wird die obige Aufgabe erfindungsgemäß durch ein derartiges Kontermittel gelöst, welches dazu dient, auf das Implantat ein Drehmoment und mit Richtungsvektor parallel zur Längsachse des Implantats zu übertragen, mit einem kontermittelseitigen Konterabschnitt, welcher ein zu dem Querschnittsprofil des implantatseitigen Konterabschnitts komplementär ausgeformtes Querschnittsprofil aufweist. Wenn der implantatseitige Konterabschnitt nach Art einer Sechskantmutter ausgeformt ist, ist erfindungsgemäß der kontermittelseitige Konterabschnitt beispielsweise als Maulschlüssel oder als Steckschlüssel mit Innensechskantprofil ausgestaltet. Auf diese Weise kann das Kontermittel als Innensechskantschlüssel ausgestaltet sein, welcher es ermöglicht, dass beim Lösen oder Anbringen von Komponenten an das Implantat mittels Herstellen oder Entfernung einer Schraubverbindung etwaige Drehmomente auf das Implantat ausgeglichen werden. Das Implantat dreht sich somit mit Vorteil nicht in unerwünschter Weise.

Das Kontermittel kann in Weiterbildung der Erfindung als Hohlzylinder zum Überstülpen über den Konterabschnitt des Grundkörpers des Implantats ausgebildet sein. Diese Ausgestaltung ermöglicht es auch, einen Bohraufsatz mit einem üblichen AO-Schaft durch das Kontermittel zu führen, um Komponenten von dem Implantat lösen zu können bzw. an dieses anbringen zu können, ohne, dass unerwünscht ein Drehmoment auf das Implantat übertragen wird.

Die Anschlussmittel gemäß dem erfindungsgemäßen Satz können an einem vorderen Ende Mittel zum lösbaren Befestigen an einem Implantat gemäß der vorgenannte Art, vorzugsweise ein zu dem hinteren Innengewinde des Implantats komplementäres Außengewinde, aufweisen, und vorzugsweise zum Durchführen durch ein Kontermittel der eingangsgenannten Art ausgebildet sind. Insbesondere können die Anschlussmittel als Bohraufsatz mit AO-Schaft ausgestaltet sein, welche zusätzlich einen Anschlag aufweisen.

Gleichermaßen wird die zugrundegelegte Aufgabe gelöst, indem das Anschlussmittel an einem vorderen Ende Mittel zum Befestigen an einer Führungsvorrichtungder vorgenannten Art , vorzugsweise ein zu dem Außengewinde der Führungsvorrichtung komplementäres Innengewinde, und mindestens einem Konterabschnitt mit einem nicht-kreisförmigen Querschnittsprofil aufweisen. Ein derart geformtes Anschlussmittel ermöglicht es erfindungsgemäß, beim Vorbohren für das endgültige erfindungsgemäße Implantat die Führungsvorrichtung, also beispielsweise den Führungsnagel, direkt mit einer Bohrvorrichtung zu verbinden, ohne dass in dieser Phase der Operation das eigentliche Implantat bereits zum Einsatz kommen muss.

Schließlich wird die auf
Aufgabe dadurch gelöst, dass mindestens ein Implantat zum Zusammenwirken mit mindestens einer der Führungsvorrichtungen und mindestens einem der Kontermittel sowie mindestens einem der Anschlussmittel ausgebildet ist. In der Praxis kann ein Satz mehrere Implantate unterschiedlicher Länge, jeweils geeignet für unterschiedliche Patienten und Krankheitsbilder, aufweisen, welche wiederum mit unterschiedlichen Führungsvorrichtungen zusammenwirken können. Beispielsweise können Führungsnägel unterschiedlicher Stärken verwendet werden, passend zu der Stärke des jeweiligen Implantats, für welche diese ein Vorbohren bewirken sollen.

Wenn der Satz erfindungsgemäß zusätzlich Anschlussmittel
enthält, kann im ersten Schritt des Vorbohrens eine Verbindung zwischen Führungsvorrichtung, also Führungsnagel zum Beispiel, und Bohrvorrichtung direkt erfolgen, ohne dass das Implantat eingebaut sein muss.

Die Erfindung wird in einer bevorzugten Ausführungsform unter Bezugnahme auf eine Zeichnung beispielhaft beschrieben, wobei weitere vorteilhafte Einzelheiten den Figuren der Zeichnung zu entnehmen sind. Funktionsmäßig gleiche Teile sind dabei mit denselben Bezugszeichen versehen.

Die Figuren zeigen im Einzelnen:
- Figur 1:: ein erfindungsgemäßes Werkzeug für eine Implantatmontage, bestehend aus einem erfindungsgemäßen Führungsnagel, einem MTV-Implantat, einem auf das MTV-Implantat aufgesteckten Steckschlüssel, sowie eine durch den Steckschlüssel geführten, mit dem MTV-Implantat verschraubten Bohreraufnahme, in seitlicher Ansicht, teilweise im Axialschnitt;
- Figur 2:: das erfindungsgemäße MTV-Implantat aus dem zusammengesetzten Werkzeug gemäß Figur 1 in separater Darstellung in seitlicher Längsansicht (a), sowie in einer Axialschnittansicht (b);
- Figur 3:: separate Darstellung des Führungsnagels gemäß der Erfindung, als Bestandteil des in Figur 1 gezeigten zusammengesetzten Werkzeugs in einer Seitenansicht in radialer Richtung;
- Figur 4:: separate Darstellung des in Figur 1 als Bestandteil des dort gezeigten zusammengesetzten Werkzeugs gezeigten Steckschlüssels als erfindungsgemäßes Kontermittel in seitlicher Ansicht in radialer Richtung (a), in axialer Draufsicht in Richtung des Pfeils B in Teil (a), sowie in einem Axialschnitt (c);
- Figur 5:: separate Darstellung der Bohreraufnahme des in Figur 1 gezeigten zusammengesetzten Werkzeugs als Ausgestaltung der erfindungsgemäßen Anschlussmittel in seitlicher Radialansicht;
- Figur 6:: erfindungsgemäßes zusammengesetztes Werkzeug in einer anderen Ausgestaltung, bestehend aus einem erfindungsgemäßen Führungsnagel als bevorzugtes Ausführungsbeispiel einer erfindungsgemäßen Führungsvorrichtung, welcher verschraubt ist mit einer erfindungsgemäßen Bohreraufnahme als bevorzugte Ausgestaltung der erfindungsgemäßen Anschlagmittel;
- Figur 7:: separate Darstellung der erfindungsgemäßen Bohreraufnahme des zusammengesetzten Werkzeugs gemäß Figur 6 in seitlicher Radialansicht, teilweise im Axialschnitt;
- Figur 8:: erfindungsgemäßer Konterschlüssel in einer Seitenansicht (a), sowie einer Draufsicht (b).

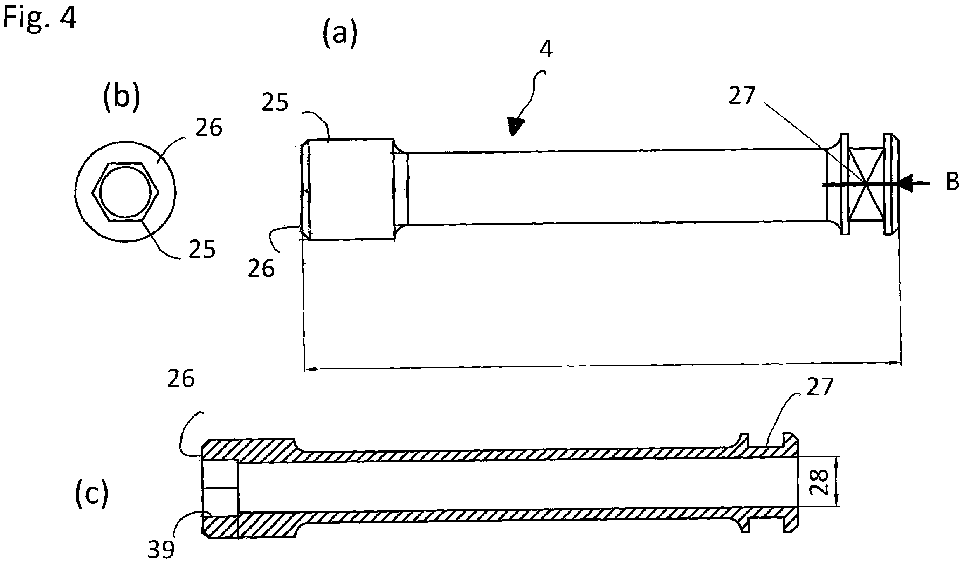

Figur 1 zeigt in seitlicher Ansicht in radialer Blickrichtung ein erfindungsgemäß zusammengesetztes Werkzeug 1 für die Implantatmontage. Das zusammengesetzte Werkzeug 1 besteht am vorderen Ende aus einer Führungsvorrichtung in Form eines Führungsnagels 2. Der Führungsnagel 2 ist weiter unten im Zusammenhang mit Figur 3 näher erläutert. Der Führungsnagel 2 ist am hinteren Ende mit einem MTV-Implantat 3 gemäß dem erfindungsgemäßen Satz für die minimal invasive transilliale Vertebralverblockung verschraubt. Das MTV-Implantat 3 gemäß dem Satz nach der Erfindung ist weiter unten im Zusammenhang mit Figur 2 näher erläutert. Auf das hintere Ende des MTV-Implantats 3 ist erfindungsgemäß ein Steckschlüssel 4 als Kontermittel aufgesetzt. Der Steckschlüssel 4 ist weiter unten im Zusammenhang mit Figur 4 näher erläutert. Durch den Steckschlüssel 4 ein Bohraufsatz 5 mit einem AO-Schaft geführt und mit dem MTV-Implantat 3 verschraubt. Der Bohraufsatz 5 ist weiter unten im Zusammenhang mit Figur 5 näher erläutert.

Das zusammengesetzte Werkzeug 1 für die Implantatmontage weist eine Gesamtlänge 6 auf, die ausreicht, um das zusammengesetzte Werkzeug 1 durch den Körper derart zu stecken, dass zumindest das vordere Ende des Führungsnagels 2 und das hintere Ende des Steckschlüssels 4 sich außerhalb des Körpers befinden, wenn das MTV-Implantat 3 am gewünschten Einbauort im Körper fixiert ist.

In Figur 2 ist das MTV-Implantat 3 gemäß dem Satz der Erfindung als Kernstück des zusammengesetzten Werkzeugs 1 für die Implantatmontage gemäß Figur 1 separat dargestellt. Dabei zeigt Teil (a) der Figur eine Seitenansicht in radialer Blickrichtung, sowie, rechts daneben, eine Aufsicht in axialer Richtung in Richtung des Pfeils 7. Teil (b) der Figur 2 zeigt einen Axialschnitt durch das MTV-Implantat 3. Das MTV-Implantat 3 weist am vorderen Ende eine axiale Bohrung mit einem Innengewinde 8 zur Aufnahme des Führungsnagels 2 auf. Am hinteren Ende ist eine axiale Bohrung mit einem Innengewinde 9, zum Einschrauben des Bohraufsatzes ausgeformt. Sowohl das Innengewinder 8, als auch das Innengewinde 9 sind Standardrechtsgewinde. In der Nähe des hinteren Endes weist das MTV-Implantat 3 eine wulstartige Erweiterung 10 mit einem Fixierungsaußengewinde 11 auf. Das Fixierungsaußengewinde 11 ist speziell für die Fixierung im Knochen ausgestaltet und umfasst zwei vollständige Windungen.

Der Bereich zwischen der wulstartigen Erweiterung 10 und dem vorderen Ende des MTV-Implantats definiert eine Nagellänge 12, welche nach den Anforderungen des operativen Eingriffs geeignet gewählt werden kann. Beispielsweise kann die Nagellänge 12 75mm betragen. Wie besonders gut in der Draufsicht im rechten Teil der Figur 2 (a) zu erkennen, ist das hintere Ende des MTV-Implantats 3 als Sechskantmutter 13 ausgeformt, durch welche die Bohrung mit dem Innengewinde 9 geführt ist. In axialer Richtung in Richtung des vorderen Endes des MTV-Implantats 3 grenzt die Sechskantmutter 13 an einen konischen Abschnitt 14 an. Der konische Abschnitt 14 überragt den zylindrischen Grundkörper 15 des MTV-Implantats 3 an der der Sechskantmutter 13 zugewandten Seite, da der Durchmesser 16 des konischen Abschnitts 14 dort größer ist als der Durchmesser 17 des zylindrischen Grundkörpers 15 des MTV-Implantats 3. Auf diese Weise entsteht eine Anlagefläche 18, die axial an die Sechskantmutter 13 angrenzt. Das MTV-Implantat 3 besteht aus rostfreiem Stahl, insbesondere nach der Norm 1.4571, und ist einteilig integral ausgeformt.

In Figur 3 ist der Führungsnagel 2 des zusammengesetzten Werkzeugs 1 für die Implantatmontage gemäß Figur 1 separat dargestellt. Bei dem
Führungsnagel 2 des erfindungsgemäßen Satzes ist das vordere Ende 19 nach Art einer geraden Pyramide spitz zulaufend ausgeformt, mit zum Schneiden beziehungsweise Bohren von Knochen ausgebildeten Kanten 20. Dies ist auch in der links in Figur 3 gezeigten axialen Draufsicht in Richtung des Pfeils 21 zu erkennen. In axialer Richtung schließt sich an das vordere Ende 19 des Führungsnagels 2 ein Vierkantabschnitt 22 an. Am hinteren Ende des Führungsnagels 2 befindet sich ein Außengewinde 23, passend zu dem Innengewinde 8 des MTV-Implantats 3. Die freie Gesamtlänge 24, die der
Führungsnagel 2 des erfindungsgemäßen Satzes im mit dem MTV-Implantat 3 verschraubten Zustand aufweist, ist so gewählt, dass mindestens der Vierkantabschnitt 22 außerhalb des Körpers liegt, wenn das MTV-Implantat 3 die gewünschte Position im Knochen des Patienten eingenommen hat.

Die Figur 4 zeigt in verschiedenen Ansichten separat den Steckschlüssel 4 des zusammengesetzten Werkzeugs 1 aus Figur 1. Wie in der Axialschnittansicht gemäß Figur 4 (c) ersichtlich, ist der Steckschlüssel 4 im Wesentlichen als Hohlzylinder ausgestaltet. Dabei ist der Durchmesser 38 der zentralen Bohrung des Steckschlüssels 4 so gewählt, dass der Bohraufsatz 5 durch den Steckschlüssel 4 gesteckt werden kann und dort relativ zu dem Steckschlüssel 4 drehbar ist. Am vorderen Ende des Steckschlüssels 4 ist innerhalb der zentralen Bohrung ein Innensechskantprofil 39 ausgeformt, welches zum Zusammenwirken mit der Sechskantmutter 13 auf Seiten des MTV-Implantats 3 ausgestaltet ist. Außerdem ist im vorderen Bereich des Steckschlüssels 4 ein Wulst 25 mit größerem Außendurchmesser angeordnet, welcher dazu führt, dass die Stirnfläche 26 am vorderen Ende des Steckschlüssels 4 einen Durchmesser hat, welcher in etwa jenem der Anlagefläche 18 des konischen Abschnitts 14 des MTV-Implantats 3 entspricht. Auf diese Weise ist das vordere Ende des Steckschlüssels 4 zum Aufstecken auf die Sechskantmutter 13 am hinteren Ende des MTV-Implantats 3 geeignet, bis die Stirnfläche 26 des Steckschlüssels 4 an der Anlagefläche 18 des MTV-Implantats 3 anliegt.

Am hinteren Ende des Steckschlüssels 4 weist der Steckschlüssel 4 ein Vierkantaußenprofil 27 zum Ansetzen eines Konterschlüssels auf. In Figur 5 ist separat der Bohraufsatz 5 des in Figur 1 gezeigten zusammengesetzten Werkzeugs 1 für die Implantatmontage dargestellt. Der Bohraufsatz 5 weist am vorderen Ende ein Außengewinde 28 zum Einschrauben in das Innengewinde 9 im MTV-Implantat 3 auf. Am hinteren Ende weist der Bohraufsatz 5 einen AO-Schaft 29 zum Anschluss an eine übliche Bohrvorrichtung auf. Der AO-Schaft 29 schließt in axialer Richtung in Richtung des vorderen Endes des Bohraufsatzes 5 einen Anschlag 30 auf, dessen Außendurchmesser zum Anlegen an die hintere Stirnfläche des Steckschlüssels 4 angepasst ist.

Die Figur 6 zeigt ein weiteres zusammengesetztes Werkzeug bestehend aus dem Führungsnagel 4 gemäß Figur 4 sowie einem Bohraufsatz 31. Der Bohraufsatz 31 ist mit dem Steckschlüssel 4 verschraubt.

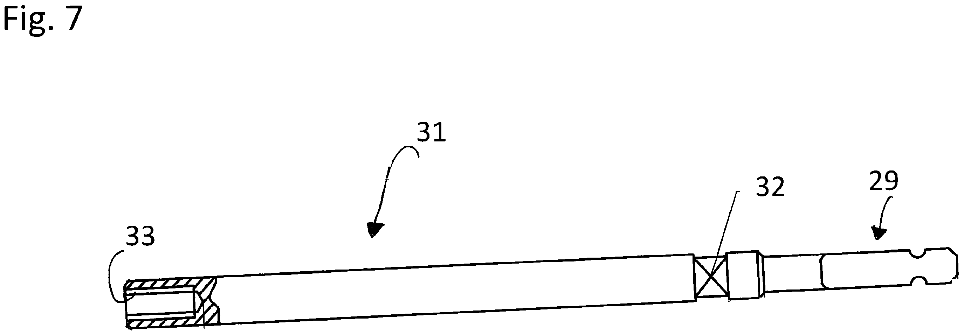

Wie in Figur 7, welche den Bohraufsatz 31 separat zeigt, zu erkennen, weist dieser am hinteren Ende einen AO-Schaft 29 zum Einspannen eine übliche Bohrvorrichtung auf. Dies kann insbesondere mittels eines Bohrfutters erfolgen. In axialer Richtung in Richtung zum vorderen Ende des Bohraufsatzes 31 weist der Bohraufsatz 31 ein Vierkantaußenprofil 32 auf zum Ansetzen eines Konterschlüssels. Im Unterschied zu dem Bohraufsatz 5 gemäß Figur 5, welcher bei dem zusammengesetzten Werkzeug 1 gemäß Figur 1 eingesetzt wird, weist der Bohraufsatz 31 am vorderen Ende eine axiale Bohrung mit einem Innengewinde 33 auf. Das Innengewinde 33 ist passend zu dem Außengewinde 23 des Führungsnagels 2 gewählt, um den Bohraufsatz 31 zur Herstellung des in Figur 6 gezeigten Werkzeugs mit dem Führungsnagel 2 verschrauben zu können.

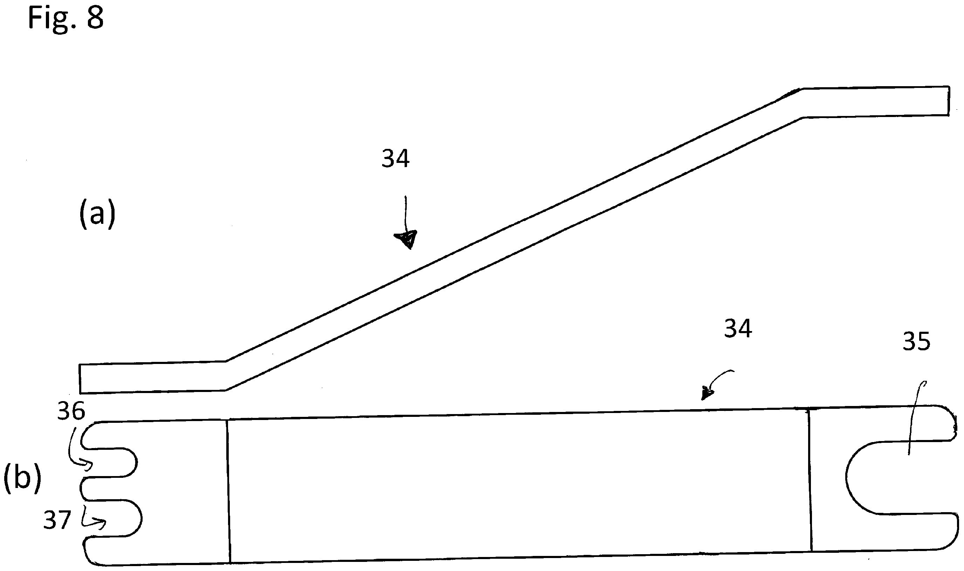

Schließlich zeigt Figur 8 in einer Seitenansicht gemäß Teil (a), sowie in einer Draufsicht gemäß Teil (b) der Figur 8 einen Konterschlüssel 34 zur Verwendung mit dem erfindungsgemäßen Satz bestehend aus dem Führungsnagel 2, dem Steckschlüssel 4 beziehungsweise mit dem Bohraufsatz 31 gemäß Figur 7.

Der Konterschlüssel 34 hat im Wesentlichen die Form einer Flachstange, welche an beiden Außenseiten jeweils um einen betragsmäßig übereinstimmenden aber entgegengesetzt orientierten Winkel abgebogen ist. In einem Endabschnitt des Konterschlüssels 34 ist ein Maulprofil 35 zum Ansetzen auf das Vierkantprofil 27 des Steckschlüssels 4 ausgeformt. Auf dem gegenüberliegenden Endabschnitt des Konterschlüssels 34 sind jeweils ein Maulprofil 36, 37 zum Ansetzen an den Vierkantabschnitt 22 des Führungsnagels 2 beziehungsweise zum Ansetzen an das Vierkantaußenprofil 32 des Bohraufsatzes 31 ausgeformt.

Zur Montage des MTV-Implantats 3 des erfindungsgemäßen Satzes im Knochen eines Patienten wird zunächst der Bohraufsatz 31 mit dem Führungsnagel 2 verschraubt, indem das Außengewinde 23 in das Innengewinde 33 eingeschraubt wird, um das in Figur 6 gezeigte zusammengesetzte Werkzeug herzustellen.

Sodann wird an geeigneten Stellen des Patienten ein Hautschnitt und eine Muskelpräparation für das Einsetzen des Führungsnagels 2 im Zielgebiet gesetzt. Anschließend wird der Führungsnagel 2 durch den Hautschnitt in den Patienten eingesetzt und durch den Knochen, in welchem das MTV-Implantat 3 zu verankern ist, geführt, mit Durchtritt durch die Haut auf der anderen Körperseite des Patienten. Dies wird mit Hilfe einer Bohrvorrichtung erwirkt, welche mit dem AO-Schaft 29 des Bohraufsatzes 31 in Wirkverbindung gebracht wird.

Der Führungsnagel 2 wird dabei auf der anderen Körperseite so weit aus dem Körper ausgeführt, dass zumindest der Vierkantabschnitt 22 des Führungsnagels 2 außerhalb des Körpers befindlich ist. Sodann wird der Konterschlüssel 34 mit dem Maulprofil 36 auf den Vierkantabschnitt 22 des Führungsnagels zum Kontern aufgesetzt. Am anderen Körperende wird der Bohraufsatz 31 gedreht, um die Schraubverbindung zwischen Außengewinde 23 und Innengewinde 33 zu lösen.

Mit dem durch den Körper und den Knochen, in welchen das MTV-Implantat 3 zu fixieren ist, befindlichen Führungsnagel 2 wird sodann zur Herstellung des zusammengesetzten Werkzeugs 1 für die Implantatmontage das MTV-Implantat 3 mit dem hinteren Ende des Führungsnagels 2 verschraubt. Dazu wird eine Schraubverbindung zwischen dem Außengewinde 23 auf Seiten des Führungsnagels 2 und dem Innengewinde 8 auf Seiten des MTV-Implantats 3 hergestellt. Für diesen Vorgang wird ein Konterschlüssel 34 in der beschriebenen Weise an den Vierkantabschnitt 22 des Führungsnagels 2 angesetzt. Andererseits wird ein Konterschlüssel mit dem Maulprofil 35 auf das Vierkantprofil 39 des Steckschlüssels 4 aufgesetzt.

Nachdem auf diese Weise das MTV-Implantat 3 mit dem Führungsnagel 2 mit Hilfe des Steckschlüssels 4 verschraubt worden ist, wird der Bohraufsatz 5 durch den Steckschlüssel 4 gesteckt, und zwar durch die Innenbohrung des Steckschlüssels 4. Außerdem wird der AO-Schaft 29 des Bohraufsatzes 5 in das Bohrfutter einer Bohrvorrichtung eingespannt. Das Außengewinde 28 des Bohraufsatzes 5 wird durch Drehen des Bohraufsatzes 5 in das Innengewinde 9 des MTV-Implantats 3 eingeschraubt, bis der Anschlag 30 des Bohraufsatzes 5 mit der hinteren Stirnfläche des Steckschlüssels 4 in Anlage kommt. Auf diese Weise ist das in Figur 1 gezeigte zusammengesetzte Werkzeug 1 zusammengesetzt, wobei der Führungsnagel 2 zumindest teilweise im Körper und im Knochen, in welchem das MTV-Implantat 3 zu verankern ist, steckt.

Nun wird das MTV-Implantat 3 durch Drehen des Bohraufsatzes 5 und durch Druck in den vom Führungsnagel 2 vorgebohrten Kanal in Körper und Knochen geführt und das Fixierungsaußengewinde 11 auf Seiten des MTV-Implantats 3 wird mit dem entsprechenden Knochen im Körper des Patienten verschraubt und auf diese Weise in diesem Knochen fixiert.

Schließlich wird ein Steckschlüssel 34 mit dem Maulprofil 35 auf das Vierkantprofil 39 des Steckschlüssels 4 aufgesetzt, um das MTV-Implantat 3 zu kontern, während der Bohraufsatz 5 aus dem MTV-Implantat 3 herausgeschraubt wird. Außerdem wird mit Hilfe des Konterschlüssels 34 und des entsprechenden Maulprofils 36 der Führungsnagel 2 aus dem im Körper fixierten MTV-Implantat 3 herausgeschraubt, wobei zeitgleich mit einem zweiten Konterschlüssel und dessen Maulprofil 35 der Steckschlüssel 4 und damit das MTV-Implantat 3 fixiert wird.

Auf diese Weise ist erfindungsgemäß ein Satz, aufweisend ein MTV-Implantat 3, einen Führungsnagel 2, einen Steckschlüssel 4, einen Bohraufsatz 5 beziehungsweise einen Bohraufsatz 31 vorgeschlagen, welcher eine Fixierung eines orthopädischen Implantats zum dauerhaften Befestigen an Knochen im Körper von Wirbeltieren und/oder Menschen auf besonders schonende Weise für den Patienten ermöglicht

### BEZUGSZEICHENLISTE

- 1: zusammengesetztes Werkzeug für Implantatmontage
- 2: Führungsnagel
- 3: MTV-Implantat
- 4: Steckschlüssel
- 5: Bohraufsatz
- 6: Gesamtlänge
- 7: axiale Blickrichtung
- 8: Innengewinde
- 9: Innengewinde
- 10: wulstartige Erweiterung
- 11: Fixierungsaußengewinde
- 12: Nagellänge
- 13: Sechskantmutter
- 14: konischer Abschnitt
- 15: zylindrischer Grundkörper
- 16: Durchmesser
- 17: Durchmesser Grundkörper
- 18: Anlagefläche
- 19: vorderes Ende
- 20: Kante
- 21: axiale Blickrichtung
- 22: Vierkantabschnitt
- 23: Außengewinde
- 24: freie Gesamtlänge
- 25: Wulst
- 26: Stirnfläche
- 27: Vierkantprofil
- 28: Außengewinde
- 29: AO-Schaft
- 30: Anschlag
- 31: Bohraufsatz
- 32: Vierkantaußenprofil
- 33: Innengewinde
- 34: Konterschlüssel
- 35: Maulprofil
- 36: Maulprofil
- 37: Maulprofil
- 38: Durchmesser
- 39: Innensechskantprofil

## Patentansprüche

1. Satz umfassend
- mindestens ein orthopädisches Implantat (3) zum dauerhaften Befestigen an Knochen im Körper von Wirbeltieren und/oder Menschen, insbesondere um damit Wirbel zu verblocken, mit einem länglichen, im wesentlichen zylindrischen Grundkörper (15), wobei es ein Fixierungsaußengewinde (11) zum Eindrehen in den Knochen aufweist, wobei der Grundkörper des Implantats (3) an einem vorderen Ende mit vorderen Befestigungsmitteln versehen ist, und wobei der Grundkörper (15) mindestens einen Konterabschnitt (13) mit einem nicht-kreisförmigen Querschnittsprofil aufweist,
- mindestens eine Führungsvorrichtung (2) zum Vorbohren eines im wesentlichen zylindrischen Kanals in Knochen im Körper von Wirbeltieren und/oder Menschen, wobei die Führungsvorrichtung (2) zur lösbaren Befestigung in Verlängerung des Grundkörpers (15) des Implantats (3) durch Zusammenwirken mit den vorderen Befestigungsmitteln (8) des Implantats (3) ausgebildet istund wobei die Führungsvorrichtung (2) mindestens einen Konterabschnitt (22) aufweist, welcher ein nichtkreisförmiges Querschnittsprofil aufweist,
- eines oder mehrere Kontermittel (4) zum Zusammenwirken mit dem Konterabschnitt (13) des Implantats (3), um auf das Implantat (3) ein Drehmoment mit Richtungsvektor parallel zur Längsachse des Implantats (3) zu übertragen, mit einem kontermittelseitigen Konterabschnitt (39), welcher ein zu dem Querschnittsprofil des implantatseitigen Konterabschnitts (13) komplementär ausgeformtes Querschnittsprofil aufweist,
- eines oder mehrere Anschlussmittel (5) mit einem Abschnitt (29) zur Herstellung einer Wirkverbindung mit einer Bohrvorrichtung, wobei die Anschlussmittel (5) an einem vorderen Ende Mittel zum lösbaren Befestigen an dem Implantat (3) aufweisen, und vorzugsweise zum Durchführen durch das Kontermittel (4) ausgebildet sind, wobei mindestens ein Implantat (3) zum Zusammenwirken mit mindestens einer der Führungsvorrichtungen (2) und mindestens einem der Kontermittel (4) sowie mindestens einem der Anschlussmittel (5) ausgebildet ist.

2. Satz gemäß Anspruch 1, **dadurch gekennzeichnet, dass** zusätzlich Anschlussmittel (31) vorgesehen sind, welche an einem vorderen Ende Mittel zum Befestigen an einer Führungsvorrichtung (2), vorzugsweise ein zu dem Außengewinde (23) der Führungsvorrichtung (2) komplementäres Innengewinde (33), und mindestens einen Konterabschnitt (32) mit einem nicht-kreisförmigen Querschnittsprofil aufweisen.

3. Satz nach einem der Ansprüche 1 oder 2, **dadurch gekennzeichnet, dass** der Grundkörper (15) des Implantats (3) an einem vorderen Ende mit einem vorderen Innengewinde (8) zur lösbaren Befestigung einer Führungsvorrichtung (2) in Verlängerung des Grundkörpers (15) versehen ist.

4. Satz nach einem der Ansprüche 1 bis 3, **dadurch gekennzeichnet, dass** das Implantat (3) mit hinteren Befestigungsmitteln, insbesondere einem hinteren Innengewinde (9) zur lösbaren Befestigung von zur Herstellung einer Wirkverbindung mit einer Bohrvorrichtung ausgeformten Anschlussmitteln (5) versehen ist.

5. Satz nach einem der Ansprüche 1 bis 4, **dadurch gekennzeichnet, dass** der Konterabschnitt (13) des Grundkörpers (15) des Implantats (3) zwischen dem Fixierungsaußengewinde (11) und dem hinteren Ende angeordnet ist.

6. Satz nach einem der Ansprüche 1 bis 5, **dadurch gekennzeichnet, dass** der Grundkörper (15) des Implantats (3) einen sich an den Konterabschnitt (13), vorzugsweise in Richtung des vorderen Endes, anschließenden, diesen an dem ihm zugewandten Ende radial überragenden und sich, vorzugsweise in Richtung des vorderen Endes, verjüngenden konischen Abschnitt (14) aufweist.

7. Satz nach einem der Ansprüche 1 bis 6, **dadurch gekennzeichnet, dass** bei dem Implantat (3) das vordere Innengewinde (8) und das hintere Innengewinde (9) eine übereinstimmende Drehrichtung aufweisen.

8. Satz nach einem der Ansprüche 1 bis 7, **dadurch gekennzeichnet, dass** die Führungsvorrichtung an einem hinteren Ende ein zu dem vorderen Innengewinde (8) des Implantats (3) komplementäres Außengewinde (23) aufweist.

9. Satz nach einem der Ansprüche 1 bis 8, **dadurch gekennzeichnet, dass** bei der Führungsvorrichtung ein vorderes Ende (19), vorzugsweise nach Art einer geraden Pyramide, spitz zulaufend ausgeformt ist, mit zum Schneiden und/oder Bohren von Knochen ausgebildeten Kanten (20).

10. Satz nach einem der Ansprüche 1 bis 9, **dadurch gekennzeichnet, dass** das Kontermittel (4) als Hohlzylinder zum Überstülpen über den Konterabschnitt (13) des Implantats (3) ausgebildet ist.

11. Satz nach einem der Ansprüche 1 bis 10, **dadurch gekennzeichnet, dass** bei dem Implantat (3) ein Kerndurchmesser des Fixierungsaußengewindes (11) größer als der Durchmesser des Grundkörpers (15) ausgestaltet ist, wobei das Fixierungsaußengewinde (11) in einem wulstartig erweiterten Abschnitt (10) des Grundkörpers (15) ausgeformt ist.

12. Satz nach einem der Ansprüche 1 bis 11, **dadurch gekennzeichnet, dass** das Fixierungsaußengewinde (11) des implantats (3) zwei komplette Gewindegänge umfasst.

13. Satz nach einem der vorhergehenden Ansprüche, **dadurch gekennzeichnet, dass** das Implantat (3) an einem hinteren Ende mit einem hinteren Innengewinde (9) zur lösbaren Befestigung von zur Herstellung einer Wirkverbindung mit einer Bohrvorrichtung ausgeformten Anschlussmitteln (5) versehen ist, wobei die Anschlussmitteln ein zu dem hinteren Innengewinde (9) des Implantats (3) komplementäres Außengewinde (28) aufweisen.

## Claims

1. Set comprising
- at least one orthopaedic implant (3) for being permanently attached to bones in the bodies of vertebrates and/or humans, in particular in order to block vertebrae, comprising an elongate, substantially cylindrical main body (15), wherein said set comprises an external fixing thread (11) for being screwed into the bone, wherein the main body of the implant (3) is provided with front attachment means on a front end, and wherein the main body (15) comprises at least one counter portion (13) which has a non-circular cross-sectional profile,
- at least one guiding device (2) for pilot-drilling a substantially cylindrical channel into bones in the bodies of vertebrates and/or humans, wherein the guiding device (2) is designed to be detachably attached in the extension of the main body (15) of the implant (3) by interacting with the front attachment means (8) of the implant (3), and wherein the guiding device (2) comprises at least one counter portion (22) which has a non-circular cross-sectional profile,
- one or more counter means (4) for interacting with the counter portion (13) of the implant (3) in order to transmit a torque to the implant (3), which torque has a direction vector that is parallel to the longitudinal axis of the implant (3), said counter means comprising a counter-means-side counter portion (39) which has a cross-sectional profile formed so as to be complementary to the cross-sectional profile of the implant-side counter portion (13),
- one or more connection means (5) comprising a portion (29) for producing an operative connection to a drilling device, wherein the connection means (5) comprise, on a front end, means for being detachably attached to the implant (3), and are preferably designed to extend through the counter means (4),
wherein at least one implant (3) is designed to interact with at least one of the guiding devices (2), at least one of the counter means (4) and at least one of the connection means (5).

2. Set according to claim 1, **characterised in that** additional connection means (31) are provided which comprise, on a front end, means for attachment to a guiding device (2), preferably an internal thread (33) that is complementary to the external thread (23) of the guiding device (2), and at least one counter portion (32) which has a non-circular cross-sectional profile.

3. Set according to either claim 1 or claim 2, **characterised in that** the main body (15) of the implant (3) is provided, on a front end, with a front internal thread (8) for detachably attaching a guiding device (2) in the extension of the main body (15).

4. Set according to any of claims 1 to 3, **characterised in that** the implant (3) is provided with rear attachment means, in particular a rear internal thread (9), for detachably attaching connection means (5) formed to produce an operative connection to a drilling device.

5. Set according to any of claims 1 to 4, **characterised in that** the counter portion (13) of the main body (15) of the implant (3) is arranged between the external fixing thread (11) and the rear end.

6. Set according to any of claims 1 to 5, **characterised in that** the main body (15) of the implant (3) comprises a conical portion (14) which adjoins the counter portion (13), preferably towards the front end, projects radially from said counter portion at the end facing said counter portion, and is tapered, preferably towards the front end.

7. Set according to any of claims 1 to 6, **characterised in that**, in the implant (3), the front internal thread (8) and the rear internal thread (9) have a congruent direction of rotation.

8. Set according to any of claims 1 to 7, **characterised in that** the guiding device comprises, on a rear end, an external thread (23) that is complementary to the front internal thread (8) of the implant (3).

9. Set according to any of claims 1 to 8, **characterised in that**, in the guiding device, a front end (19) is formed so as to be tapered, preferably in the manner of a right pyramid, having edges (20) designed for cutting and/or drilling bones.

10. Set according to any of claims 1 to 9, **characterised in that** the counter means (4) is designed as a hollow cylinder for being placed on the counter portion (13) of the implant (3).

11. Set according to any of claims 1 to 10, **characterised in that**, in the implant (3), a core diameter of the external fixing thread (11) is designed so as to be larger than the diameter of the main body (15), the external fixing thread (11) being formed in a portion (10) of the main body (15) that is extended in the manner of a bead.

12. Set according to any of claims 1 to 11, **characterised in that** the external fixing thread (11) of the implant (3) comprises two complete turns.

13. Set according to any of the preceding claims, **characterised in that** the implant (3) Is provided, on a rear end, with a rear internal thread (9) for detachably attaching connection means (5) formed to produce an operative connection to a drilling device, the connection means comprising an external thread (28) that is complementary to the rear internal thread (9) of the implant (3).

## Revendications

1. Ensemble comprenant
- au moins un implant (3) orthopédique destiné à être fixé de manière durable sur des os dans le corps d'animaux vertébrés et/ou d'hommes, en particulier afin de bloquer des vertèbres, avec un corps de base (15) allongé sensiblement cylindrique, lequel présente un filetage extérieur de fixation (11) destiné à être introduit par rotation dans l'os, dans lequel le corps de base de l'implant (3) est pourvu, au niveau d'une extrémité avant, de moyens de fixation avant, et dans lequel le corps de base (15) présente au moins une section de blocage par contre-écrou (13) avec un profil de section transversale de forme non circulaire,
- au moins un dispositif de guidage (2) destiné à percer au préalable un canal sensiblement cylindrique dans des os dans le corps d'animaux vertébrés et/ou d'hommes, dans lequel le dispositif de guidage (2) est réalisé aux fins de la fixation amovible dans le prolongement du corps de base (15) de l'implant (3) par coopération avec les moyens de fixation (8) avant de l'implant (3) et dans lequel le dispositif de guidage (2) présente au moins une section de blocage par contre-écrou (22), qui présente un profil de section transversale de forme non circulaire,
- un ou plusieurs moyens de blocage par contre-écrou (4) destinés à coopérer avec la section de blocage par contre-écrou (13) de l'implant (3) afin de transmettre sur l'implant (3) un couple de rotation avec un vecteur directionnel parallèle à l'axe longitudinal de l'implant (3), avec une section de blocage par contre-écrou (39) située du côté du moyen de blocage par contre-écrou, laquelle présente un profil de section transversale formé de manière complémentaire au profil de section transversale de la section de blocage par contre-écrou (13) située du côté de l'implant,
- un ou plusieurs moyens de raccordement (5) avec une section (29) destinés à établir une liaison active avec un dispositif de perçage, dans lequel les moyens de raccordement (5) présentent au niveau d'une extrémité avant des moyens destinés à être fixés de manière amovible au niveau de l'implant (3), et de préférence destinés à être guidés à travers le moyen de blocage par contre-écrou (4),
dans lequel au moins un implant (3) est réalisé afin de coopérer avec au moins un des dispositifs de guidage (2) et au moins avec un des moyens de blocage par contre-écrou (4) ainsi qu'avec au moins un des moyens de raccordement (5).

2. Ensemble selon la revendication 1, **caractérisé en ce que** sont prévus en supplément des moyens de raccordement (31), qui présentent, au niveau d'une extrémité avant, des moyens de fixation à un dispositif de guidage (2), de préférence un filetage intérieur (33) complémentaire au filetage extérieur (23) du dispositif de guidage (2), et au moins une section de blocage par contre-écrou (32) pourvue d'un profil de section transversale de forme non circulaire.

3. Ensemble selon l'une quelconque des revendications 1 ou 2, **caractérisé en ce que** le corps de base (15) de l'implant (3) est pourvu au niveau d'une extrémité avant d'un filetage intérieur (8) avant destiné à fixer de manière amovible un dispositif de guidage (2) dans le prolongement du corps de base (15).

4. Ensemble selon l'une quelconque des revendications 1 à 3, **caractérisé en ce que** l'implant (3) est pourvu de moyens de fixation arrière, en particulier d'un filetage intérieur (9) arrière destiné à fixer de manière amovible des moyens de raccordement (5) formés afin d'établir une liaison active avec un dispositif de perçage.

5. Ensemble selon l'une quelconque des revendications 1 à 4, **caractérisé en ce que** la section de blocage par contre-écrou (13) du corps de base (15) de l'implant (3) est disposée entre le filetage extérieur de fixation (11) et l'extrémité arrière.

6. Ensemble selon l'une quelconque des revendications 1 à 5, **caractérisé en ce que** le corps de base (15) de l'implant (3) présente une section (14) conique se raccordant à la section de blocage par contre-écrou (13), de préférence en direction de l'extrémité avant, dépassant de manière radiale de ladite section au niveau de l'extrémité tournée vers la section de blocage par contre-écrou et se rétrécissant de préférence en direction de l'extrémité avant.

7. Ensemble selon l'une quelconque des revendications 1 à 6, **caractérisé en ce que** dans le cas de l'implant (3), le filetage intérieur avant (8) et le filetage intérieur arrière (9) présentent une direction de rotation qui coïncide.

8. Ensemble selon l'une quelconque des revendications 1 à 7, **caractérisé en ce que** le dispositif de guidage présente au niveau d'une extrémité arrière un filetage extérieur (23) complémentaire au filetage intérieur avant (8) de l'implant (3).

9. Ensemble selon l'une quelconque des revendications 1 à 8, **caractérisé en ce que** dans le cas du dispositif de guidage une extrémité avant (19) est formée de manière à converger en pointe, de préférence à la manière d'une pyramide droite, avec des arêtes (20) réalisées pour le découpage et/ou le perçage d'os.

10. Ensemble selon l'une quelconque des revendications 1 à 9, caractérisé en ce en ce que le moyen de blocage par contre-écrou (4) est réalisé sous la forme d'un cylindre creux à retourner sur la section de blocage par contre-écrou (13) de l'implant (3).

11. Ensemble selon l'une quelconque des revendications 1 à 10, **caractérisé en ce que** dans le cas de l'implant (3), un diamètre central du filetage extérieur de fixation (11) est plus grand que le diamètre du corps de base (15), dans lequel le filetage extérieur de fixation (11) est formé dans une section (10) élargie à la manière d'un bourrelet du corps de base (15).

12. Ensemble selon l'une quelconque des revendications 1 à 11, **caractérisé en ce que** le filetage extérieur de fixation (11) de l'implant (3) comprend deux pas de filetage complets.

13. Ensemble selon l'une quelconque des revendications précédentes, **caractérisé en ce que** l'implant (3) est pourvu, au niveau d'une extrémité arrière, d'un filetage intérieur arrière (9) destiné à fixer de manière amovible des moyens de raccordement (5) formés afin d'établir une liaison active avec un dispositif de perçage, dans lequel les moyens de raccordement présentent un filetage extérieur (28) complémentaire au filetage intérieur arrière (9) de l'implant (3).
